# EUROPEAN PATENT SPECIFICATION

(11) **EP 0 749 779 B1**
(45) Date of publication and mention of the grant of the patent: **05.01.2000**
(21) Application number: 95304421.1
(22) Date of filing: 23.06.1995
(51) Int. Cl.: B01J 23/89, C07C 5/333

(54) **Catalyst composite for dehydrogenation of paraffins to monoolefins and method for the preparation thereof**
Zusammengesetzter Katalysator für die Dehydrierung von paraffinen in Monoolefinen und Verfahren für seine Herstellung
Catalyseur composite pour la deshydrogénation des paraffines en monooléfines et méthode pour le préparer

(43) Date of publication of application: 27.12.1996
(73) Proprietor: INDIAN PETROCHEMICALS CORPORATION LIMITED, District Vadodara 391346 Gujarat (IN)
(72) Inventor: Dongara, Rajeshwer, c/o Indian Petrochemicals, District Vadodara -391 346 Gujarat (IN); Basrur, Arun Gurudath, c/o RESEARCH CENTRE, District Vadodara -391 346 Gujarat (IN); Gokak Dattatraya Tammannashastri, c/o Indian Petro, District Vadodara -391 346 Gujarat (IN); Rao, Karumanchi Venkateshwara, c/o Indian Petro, District Vadodara -391 346 Gujarat (IN); Krishnamurthy, Konda Ramaswamy, c/o Indian Petro, District Vadodara -391 346 Gujarat (IN); Bhardwaj, Ishwar Singh, c/o RESEARCH CENTRE, District Vadodara -391 346 Gujarat (IN)
(74) Representative: Marshall, Monica Anne

(56) References cited:
- EP-A- 0 183 861
- US-A- 3 951 868
- US-A- 4 078 743
- US-A- 4 136 064
- US-A- 4 197 416

## Description

The present invention relates to a novel catalytic composite useful in the dehydrogenation of normal paraffins to the respective mono olefins and to a method for the preparation thereof. More specifically, the invention relates to a catalyst composite exhibiting high activity, stability and selectivity wherein the active elements are deposited in a pre-determined manner within the spatial geometry of the carrier therefor.

Dehydrogenation of paraffins, specifically C₂-C₂₀ paraffins, is an important petrochemical process through which a number of products useful in day to day life, such as plastics, synthetic rubber and detergents are manufactured. Furthermore, dehydrogenation of naphthenes and paraffins are the most facile reactions during catalytic reforming processes, practiced worldwide, for the production of aromatics (BTX) and high octane gasoline.

The dehydrogenation of n - paraffins especially in the range C₁₀ to C₁₆ is a reaction of prime importance particularly to the detergent industry for the manufacture of detergents. The dehydrogenation comprises a series of complex reactions involving parallel reactions accompanied by cracking, isomerization and coking reactions. The desired product is n-mono olefin which is formed in the first step of this series of reactions. Hence, selectivity is of paramount consideration. At the same time, there is a drawback: the life of most dehydrogenation catalysts is relatively short.

Towards maximising selectivity and increasing catalyst life-span, it is conventional to attempt to suppress accompanying reactions by thermodynamically limiting per pass conversion by the application of pressure. However, it is the overall performance in terms of activity, selectivity and stability as exhibited by the catalyst that actually dictates the optimum feasibility of this reaction. This activity can be defined in terms of extent of per pass conversion as "the moles of paraffin converted per mole of paraffin fed", in terms of selectivity as "moles of n-mono olefin formed per mole of paraffin converted" and in terms of stability as "duration for which required activity and selectivity are maintained during the course of the reaction". Hence, it is the chemical components employed and the method of preparation of the catalytic composite which are responsible for any unique physico-chemical properties in the catalyst which enable it to evince these virtues. Catalyst preparation thus plays a key role.

Most catalytic compositions used to date for the dehydrogenation of paraffins consist of the following metals or combinations thereof, supported on a suitable porous, high surface area support such as gamma Al₂O₃:
1. Noble metal (Pt,Re,Pd,Ir,Au,Os).
2. Noble metal + Group IV A metal (Ge,Sn,Pb).
3. Noble metal + Group IV A metal + Group III A metal (Ga, In, Tl).
4. Noble metal + Other Group VIII metals, (Fe,Co,Ni) and/or alloys thereof.
5. 3 above + Group III A metal or Group III B metal (Sc,Y,La,Ac) as primary active agents, with Group II B metal (Zn,Cd,Hg) as optional secondary activating agents.
6. Noble metal + Group V A metal (As,Sb,Bi) or VI A element (S,Se,Te).
7. 1, 2, 3, 4, 5 or 6 above + Group VII A element, viz halogen in combined form.
8. 1, 2, 3, 4, 5, 6, 7 above + Group I A metal or Group II A metal, viz alkali or alkaline earth metals.
9. 1, 2, 3, 4, 5, 6, 7 or 8 above + Sulfur.
The references to Groups are based on the CAS version of the Periodic Table.

Indian Patent Specification No. 41,667 discloses a Pt/Al₂O₃ catalyst with combined halogen less than 8 weight percent calculated on an elemental basis suitable for dehydrogenation, reforming hydrogenation, hydrocracking and oxidation applications. U. S. Patents 2,479,109 and 2,479,110 also disclose a catalyst of like composition.

U. S. Patent No. 2,602,772 discloses addition of oxide of alkali metal or alkaline earth metal or Mg not exceeding 1 weight percent to a Pt/Al₂O₃ catalyst containing 0.1 - 8 weight percent combined halogen. The effect of Mg is reportedly lesser coke formation.

U. S. Patent 2,930,763 discloses a catalyst composition consisting of Pt 0.1 - 1.0 weight percent, combined halogen (calculated on elemental basis) 0.1 - 1.0 weight percent, and alkali metal 0.01 - 1 weight percent for use in reforming applications in which dehydrogenation is one of the predominant reactions.

Addition of elements of Group IV A or Group III A are disclosed in the following patents. U. S. Patent 3,531,543 discloses a catalyst composition for dehydrogenation applications containing Pt, Sn, alkali metal and combined halogen, wherein the alkali metal is added to the support in a first step to yield a support like lithiated Al₂O₃. The purpose of alkali metal addition is to obtain a relatively neutral support.

U. S. Patent 3,745,112 discloses a catalyst primarily for reforming applications of a similar composition to that disclosed in U. S. Patent 3,531,543 wherein the role of the alkali metal is described as killing of the acidic function of the catalyst. Sn is described as a good promotor. U. S. Patent 3,909,451 also describes a catalyst of similar composition for dehydrogenation wherein the combined halogen content, as calculated on an elemental basis, is less than 0.2 weight percent. Similarly, U. S. Patents 4,329,258 and 4,363,721 describe catalysts containing Pt, Sn and an alkali metal and combined halogen wherein the atomic ratio of alkali metal to Pt is in the range 0.2 to 10.

U. S. Patent 3,892,657 discloses a catalyst consisting of Pt, In and one of Ge, Sn or Pb along with combined halogen, the halogen content, as calculated on an elemental basis, varying from 0.1 weight percent for dehydrogenation application up to 3.5 weight percent for reforming applications and up to 10 weight percent for isomerisation applications. In is described as a good promotor when the atomic ratio of In : Pt is 0.1 : 1 to 1:1. A combination of Pt, Sn, In and Cl or Pt and one of Ge, Sn, Pb plus In is described as suitable for reforming reactions while a combination of Pt, In and alkali or alkaline earth metals is stated to be suitable for dehydrogenation. The combination of Pt, Sn, In and an alkali/alkaline earth element is not specifically disclosed.

Indian Patent Specification No. 128185 discloses a catalyst consisting of Pt, Ge and an alkali metal or alkaline earth metal on alumina for dehydrogenation applications. Indian Patent Specification No. 128349 describes a catalyst consisting of Pt, Sn and Ge on an alumina carrier. Indian Patent Specification No. 140805 discloses the addition of an alkali metal or alkaline earth metal in an amount of from 0.01 - 5 weight percent to a catalyst of composition as described in Indian Patent No, 128349 and shows the beneficial effect of this addition. The alkali/alkaline earth component is preferably added after impregnation of Pt, Sn and Ge.

Indian Patent Specification No. 145594 describes a catalyst composition consisting of Pt 0.2-1%, one of Ga, In,Tl, 0.2 - 1.0%, an alkali or alkaline earth element 0.2 to 2 weight percent and combined halogen 0.01 to 0.1 weight percent. This patent shows the superiority of such composition over prior art catalysts consisting of Pt/Al₂O₃ with alkali or alkaline earth elements with optionally, As or Pb as promotors.
British Patent No. 1,499,297 discloses a catalyst similar in composition to that disclosed in Indian Patent Specification No. 145594 for dehydrogenation applications wherein the alkali metal is preferably Li or K, and its atomic ratio in relation to Pt is up to 10 with combined halogen in the range 0.01 - 0.1 weight percent. Such composition is described as resulting in better selectivity and stability.

Indian Patent Specification No. 163412 also discloses a catalyst of composition similar to that disclosed in British Patent 1499 297 and Indian Patent Specification No. 145594. However, it claims that combined halogen content greater than 0.2% and an atomic ratio of alkali metal to Pt greater than 10 results in improved activity and selectivity.

Indian Patent Specification No.165513 describes a catalytic composition consisting of Pt, one of Group IV A metal selected from Ge, Sn or Pb and an alkali or alkaline earth metal in an amount whereby the atomic ratio of this latter metal to Pt is greater than 10, and a combined halogen content exceeding 0.2 weight percent. Such composition is shown to exhibit better activity and selectivity than prior art catalysts with halogen content of less than 0.2 weight percent.

Indian Patent Specification No. 166585 discloses use of bifurcated alkali component such as a combination of Li and K to a catalyst of composition similar in other respects to that disclosed in Indian Patent Specification No. 165513.

Indian Patent Specification No. 161974 discloses a catalyst for dehydrogenation applications consisting of Pt, Sn, In, an alkali or alkaline earth component and combined halogen, wherein the atomic ratio of In: Pt is greater than 1. The support is preferably an Sn - Al₂O₃ support. The patent discloses the promoting action of In.
U. S. Patent 3,632,661 discloses a catalyst consisting of Pt or Pd 0.1 - 5% along with Fe 0.01-10% or oxides/alloys thereof as promotors. The catalyst includes optionally Group I B metals as secondary promotors 0.002-5% or one of Co or Zn 0.1 - 4% with 0.2 - 2 % weight percent alkali/alkaline earth metals on a near neutral carrier. The method avoids the use of halogenated salts for impregnation and preferably impregnates the promotor prior to the noble metal.

U. S. Patents 2,814,599 and 2,914,464 describe catalysts containing one or more of Ga, In, Sc, Y, La, Tl and Ac as primary activating agents along with the optional addition of one or more of Hg, Zn or Cd as secondary activating agents for improved reforming activity.

Indian Patent Specification No. 136459 discloses a catalyst used in the dehydrogenation step in the production of aryl substituted n-paraffins which consists of Pt 0.05 - 5 weight percent, an alkali or alkaline earth metal component such as Li 0.01 - 1.5 weight percent and one of Group V A or Group VI A elements, viz As, Sb, Bi, S, Se, Te, preferably As, 0.01 - 1 weight percent.

The present invention discloses a novel catalyst composite which includes Fe alongwith Pt, Sn, In, Li and Cl. The novel catalyst composite of the present invention has better activity, selectivity and stability than the catalyst composites of the prior art and hence improves its overall performance in a reaction.

It is, therefore, the basic object of the present invention to provide a novel catalyst composite for use in the dehydrogenation of paraffins to monoolefins in which the catalyst framework exhibits a predetermined distribution of the active elements within the spatial geometry of the catalyst particle.

A further object is the provision of a method for the preparation of such a catalyst composite which permits the distribution of active elements within the spatial geometry of the catalyst according to a pre-determined requirement.

Another object of the invention is to provide a catalyst having the ability to control within the spatial geometry of the catalyst frame work a desired distribution of the active elements of the catalyst.

Accordingly, the present invention provides a novel catalyst composite for use in the dehydrogenation of paraffins to the corresponding monoolefins, said composite incorporating within its spatial geometry on a percentage by weight basis a predetermined concentration gradient of the following active elements:
from 0.1 to 5.0% of a noble metal;
from 0.1 to 5.0 % of a metal of Group IV A;
from 0.1 to 6.0 % of a metal of Group III A;
from 0.1 to 10.0 % of an alkali or alkaline earth metal element;
from 0.01 to 10.0% of a halogen; and
from 0.1 to 5.0 % of a metal of Group VIII selected from Fe, Co and Ni provided on a high surface area mesoporous support.

According to a preferred embodiment, the catalyst composite of this invention consists of:
Platinum as the noble metal;
Tin (Sn) as the metal of Group IV A;
Indium (In) as the metal of Group III A;
Lithium (Li) as the alkali or alkaline earth metal element;
Chlorine (Cl) in combined form as the halogen; and
Iron (Fe) as the metal of Group VIII.

Preferably, Cl⁻ is present in an amount of from 0.05 % to 0.1 % by weight calculated on an elemental basis and Fe is present in an amount of approximately 0.2 % by weight.

The mesoporous support is preferably a spheroidal gamma alumina support having a diameter of 1.4 to 2.0 mm, a surface area in the range of from 120 to 250 m²/g, a mesoporous pore distribution, a water adsorption capacity in the range of from 1.4 to 2.5 ml/g, a gamma crystallinity of from 60 to 80% and a bulk density of from 0.27 to 0.6 g/ml, preferably 0.3 gm/ml. Optionally the support also comprises 0.1-5.0 wt% Fe. In addition, the support should evince sound mechanical strength in terms of crush strength and loss on attrition.

The present invention also provides a process for the preparation of a novel catalyst composite incorporating a predetermined concentration gradient of active elements within its spatial geometry for use in the dehydrogenation of paraffins to the corresponding monoolefins which comprises incorporating on a percentage by weight basis within a high surface area mesoporous support:
from 0.1 to 5.0% of a noble metal;
from 0.1 to 5.0 % of a metal of Group IV A;
from 0.1 to 6.0 % of a metal of Group III A;
from 0.1 to 10.0% of an alkali or alkaline earth metal element;
from 0.01 to 10.0% of a halogen; and
from 0.1 to 5.0 % of a metal of Group VIII selected from Fe, Co and Ni drying the composite in which said active elements have been incorporated; and
subjecting the dried composite to at least one calcination step.

According to the method of the present invention, the active elements can be incorporated simultaneously in combination into the mesoporous support in a single step or they may be incorporated stagewise employing any conventional procedure for the purpose. For instance, the active elements can be incorporated individually or in combination into the support during the preparation of the latter. Alternatively, such elements can be incorporated by impregnation of the finished support therewith, again individually or in combination.

In accordance with a specific embodiment, the noble metal, the metal of Group IV A, the metal of Group III A, the alkali or alkaline earth metal and the metal of Group VIII can be incorporated into an alumina support by co-precipitation or co-gellation during the sol state in the preparation of such support.

In an alternative embodiment, the metals identified can be impregnated into finished support. Such impregnation can be effected employing all the metals simultaneously or successively in any order.

For such impregnation, any conventional method may be employed such as equilibrium adsorption, incipient wetness, spraying, deposition as a film from the vapour state, co-precipitation, co-gellation or a combination of any of these. The choice of impregnation procedure will determine the formation of the desired concentration gradient of the active elements with the catalyst composite. The chosen procedure can also result in a composite having a heterogeneous or shell type deposition of elements therein or a homogeneous or uniform deposition of elements therein.

Preferably the noble metal, essentially platinum, is incorporated into the support simultaneously with the Group IV A metal, essentially tin.

Alternatively, the Group IV A metal is incorporated into the support in a separate step prior to incorporation of the other elements.

The Group III A metal, essentially indium, is not incorporated after the noble metal has been incorporated into the support.

The alkali or alkaline earth metal, essentially lithium is preferably incorporated into the support prior to or simultaneously with the other elements.

Incorporation of the active elements into the finished support by impregnation is conveniently effected employing an aqueous solvent, an organic solvent or mixture of the two, in the presence of anions. Such anions are preferably acidic and in particular chloride anions in a concentration range from 0.1% to 15% by weight preferably 5% to 10% by weight. This range is maintained in order to achieve the desired distribution of active elements within the support framework for the catalyst.

In accordance with a preferred feature, the alumina support contains iron as the Group VIII metal which iron has been incorporated into the support at the sol stage of its preparation or in the first impregnation step.

Preparatory to impregnation, the aqueous and organic solutions employed for the purpose are preferably heated to a temperature of from 40°C to 70°C for a minimum of 30 minutes. Thereafter, the solutions are cooled to a temperature in the range of from 5°C to 40°C and maintained at this range during the impregnation step. This procedure yields a catalyst with uniform dispersion of elements therein and ensures minimal loss of the impregnated support which would otherwise occur by mechanical fracture of the support as a result of the heat of adsorption of the impregnating solution.

The quantum of the solution employed for impregnation by the incipient wetness technique is from 5% to 30% by weight in excess of the water adsorption capacity of the support measured according to IS 9700 - 1981.

The procedure found most convenient for incorporation of the active elements into the support is spraying of a solution of such elements on to the support at a controlled rate while rotating the support at a predetermined angle of inclination with respect to the spray.

After incorporation therein of the active elements, the catalyst composite is preferably dried in a dust-free environment at ambient temperature for from 0.5 to 6 hours and thereafter in a flow of purified air at a temperature from 80°C to 170°C for 4 to 12 hours.

The calcination of the dried catalyst composite can be effected in one or more stages at a temperature in the range of from 400°C to 650°C for a period of from 4 to 16 hours in an environment of circulating dry air.

According to a further feature of the invention, the calcined catalyst composite is subjected to dehalogenation treatment preferably by employing a counter current stream in order to reduce its halogen content to 0.01% to 5%, preferably 0.05% to 0.1%, by weight. The dehalogenated composite is then washed, dried and further calcined whereafter it is ready for use in the dehydrogenation of C₁₀ to C₁₄ n-paraffins.

A preferred washing medium for the dehalogenated composite is demineralised water with the washing effected at a temperature of 10°C to 80°C for a period of 0.5 to 5 hours. The drying and calcination are effected in the manner identified above.

It has been found convenient to dehalogenate the catalyst composite by subjecting it to steaming employing a 20 : 80 steam-air mixture at a temperature of from 400°C to 550°C.

Alternatively, the composite can be dehalogenated by treating it at a temperature of from 10°C to 80°C for a period of from 0.5 to 10 hours with an aqueous solution of a weak base or salt thereof or with an aqueous solution of an organic or inorganic compound which undergoes hydrolysis to release such weak base at the temperature mentioned. This alternative dehalogenation treatment is preferred and the preferred temperature thereof is 40°C to 80°C.

The invention will be described in greater detail with reference to the accompanying drawings and examples that follow.
Fig. 1 Brings out the effect of Fe in the catalyst formulation.
Fig. 2 Comparison of activities of catalyst formulation prepared.
Fig. 3 Concentration profile of Pt, Sn, In, in catalyst formulation D.
Fig. 4a Effect of Cl on concentration profile of Sn in catalysts prepared by incepient wetness.
Fig. 4b Effect of Cl content and method of impregnation on concentration profile of Pt, Sn and Cl.
Fig. 4c. Effect of Cl content of impregnating solution on concentration profile of In and Fe.
Fig. 5 Effect of residual Cl content of catalyst on stability.

In such examples, unless otherwise specified, a chloride content of between 8 to 10 weight percent is maintained in the impregnating solution whenever spraying is employed as the preferred manner of impregnation.

### EXAMPLE - I

### Preparation of Catalyst Composite of Formulation A - The benefit of including Fe in the catalyst formulation.

Employing spray procedure in a two-step impregnation of a spheroidal alumina support, a catalyst composite according to the present invention was prepared having the following composition by weight:
- Pt: 0.4%
- Fe: 0.2%
- Sn: 0.5%
- In: 0.4%
- Li: 0.6%
- Cl: 0.07%

The alumina support employed had an average particle diameter of 1.85 mm with 68% gamma crystallinity, a BET surface area of 170 m²/g, a water adsorption capacity (WAC) of 1.8 ml/g, mesoporous distribution of pores, a bulk density of 0.31 g/ml, and a Fe content of 0.2wt % as a solution of Fe [N0₃]₃ which was incorporated into the support at the sol stage in its preparation.

In the first step of impregnation, a solution of LiNO₃ was employed to impregnate the support whereafter the support thus impregnated was dried and calcined. Pt, Sn and In were impregnated in a second step by spraying it with a second solution containing H₂PtCl₆, SnCl₂, In[NO₃]₃ and HCl. The re-impregnated support was once again dried and calcined and then subjected to a de-halogenation step in order to reduce the chlorine content thereof to 0.07% by weight. This catalyst exhibits a monoolefin selectivity of 89-90%.

### EXAMPLE - II (Comparative)

### Preparation of Catalyst Composite of Formulation B

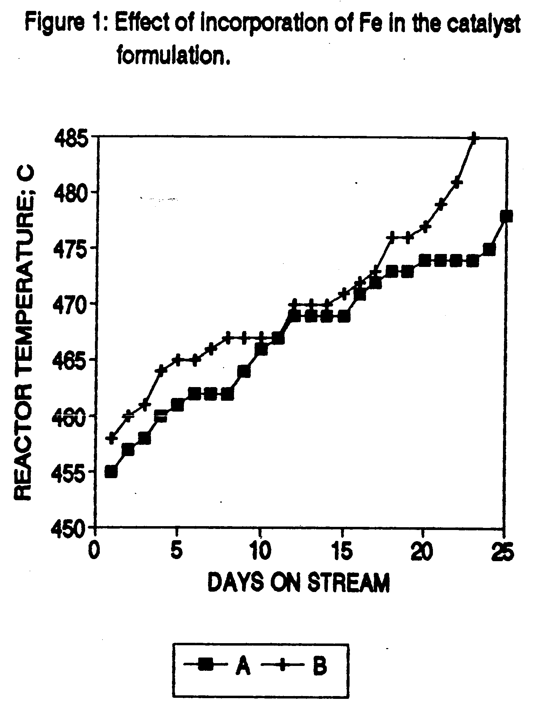

Formulation B was prepared using the same support as for Formulation A the only difference being that Fe was not included in the formulation. The results are presented in figure 1. It is clear that incorporation of Fe increases both the activity [temperature for a given paraffin conversion is lower than in catalyst B] and stability [at 25 days on stream the reactor temperature is 10°C lower than for catalyst B] of the catalyst.

### EXAMPLE III

### Preparation of Catalyst Composite of Formulation C - The importance of addition of Pt and Sn together.

Catalysts of composition similar to that in Example I, Formulation A, were prepared by a different method of impregnation wherein Fe was incorporated at sol stage, then Sn and Li were impregnated onto the alumina support in a first step by spraying an aqueous mixture of SnCl₂ in 5% HCl and LiNO₃ onto the support as per the method described in this patent. This was followed by a drying and calcination step followed by incorporation of Pt and In together in a second step also by spraying of a mixture of the solutions of their salts as described in this patent. The catalyst was dried and calcined. It was then subjected to a dehalogenation step and the Cl⁻ content reduced to 0.08 wt. %.

The results of evaluation of this catalyst formulation vis a vis Formulation A showed that the initial activity of both catalysts is identical but Formulation C tends to deactivate rapidly due to coking [fall in activity is about 1.25 times that of Formulation A]. The results show that incorporation of the other active elements, especially Sn, prior to incorporation of Pt results in relatively poor stability.

### EXAMPLE IV

### Preparation of Catalyst Composite of Formulation D

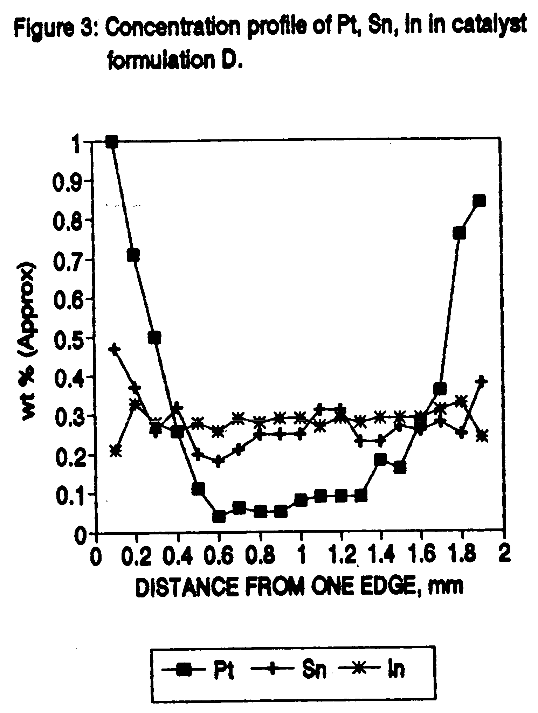

The support used was identical to that used in the case of formulation A. Final compositions too were similar. The method of impregnation however is two step, Li, Sn and In were incorporated in the first step by the spray method described in this patent. This was followed by drying and calcination prior to loading of Pt from a solution of H₂PtCl₆ by the equilibrium adsorption method wherein the solid/solution ratio was maintained at 1 : 7, additional Cl⁻ as HCl was not added to this solution. The contact time was 24h, Pt uptake from solution was 99 weight percent. This was followed by drying and calcination. This formulation results in a shell type catalyst wherein Pt is restricted to an outer shell, approximately 300 nm thick, of the catalyst spheroid of average diameter 1.85 mm. The other active elements are uniformly distributed. The interior of the catalyst particle contains very little of Pt. The EDAX (Energy Dispersive Analysis Of X-Rays) results of the profiles of various active elements are shown in Figure 3.

### EXAMPLE V

### Preparation of Catalyst Composite of Formulation E

Formulation E is similar in composition to formulation D. In its method of preparation the steps of formulation D have been reversed. Pt as H₂PtCl₆, in aqueous HCl amounting to 1 wt. % Cl⁻, was incorporated in the first step by the equilibrium adsorption technique. The catalyst was dried and calcined. The other elements Sn, In, Li were added by spraying in a second step. Followed by drying, calcination and dehalogenation.

### EXAMPLE VI

### Preparation of Catalyst Composite of Formulation F

Formulation F was prepared in a manner similar to formulation E, except that the other elements, Li, Sn and In were impregnated by spraying in a first step followed by drying and calcination. The Pt along with 1% Cl⁻ as HCl was incorporated by equilibrium adsorption in a second step. The catalyst was dried, calcined and dehalogenated by the preferred method as cited in this patent.

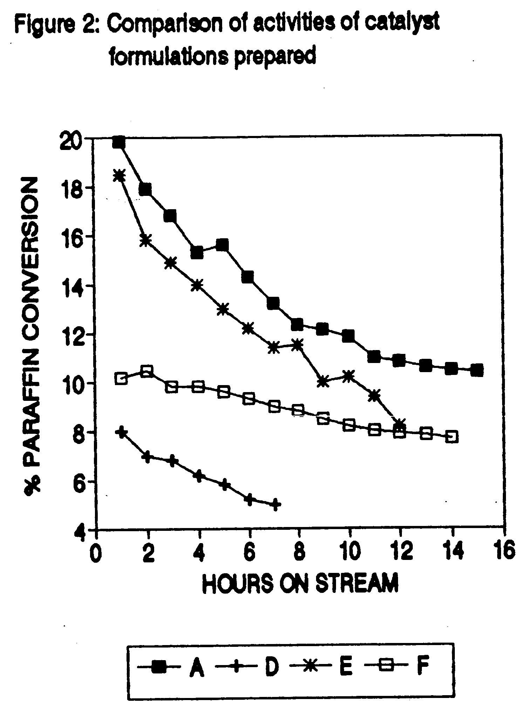

Results of C10-C13 n-paraffin dehydrogenation over these two catalysts are presented in figure 2. The results for formulation A which is prepared by the preferred method as disclosed in this patent is also included in this figure for comparison. Catalyst formulation A is undoubtedly the superior of the four followed by E. The performance of formulations F and in particular D is poor. These catalysts were prepared by addition of Sn, In and Li to Al₂O₃ by spraying and incorporating Pt in a second step by equilibrium adsorption method. The subtle difference between the latter two is the chloride content of the impregnating solution. In the case of formulation D additional Cl⁻ in the form of HCl was not added to the impregnating solution whereas in case of F, 1 weight percent of Cl⁻ was added as HCl. In spite of its poor catalytic activity the method of preparation of formulation D in particular, provides a means for distribution of the active element/s viz. Pt selectively in the shell region as shown in Figure 3.

### EXAMPLE VII

### Preparation of Catalyst Composite of Formulations G, H, I, J, K, L

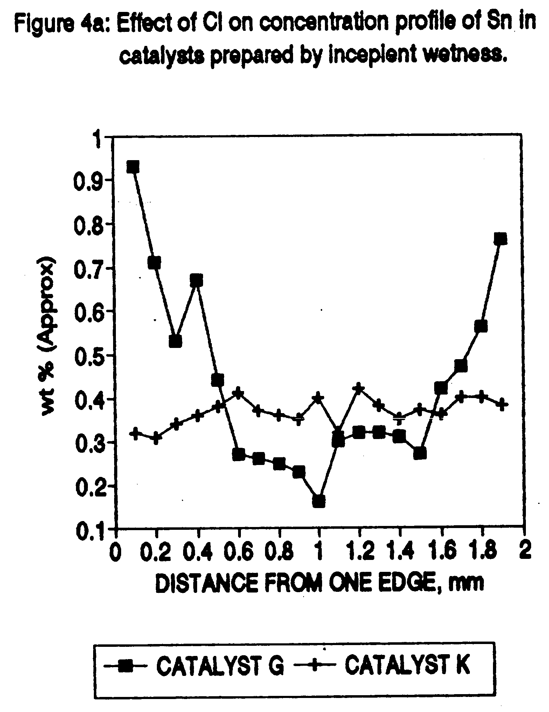

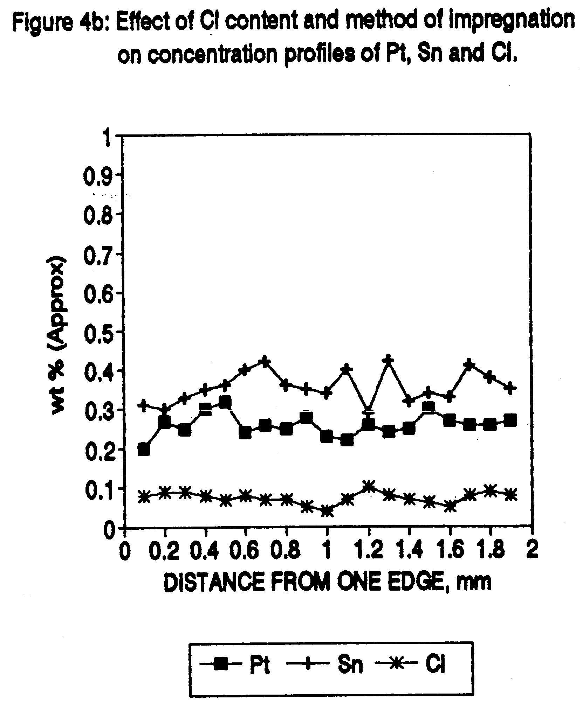

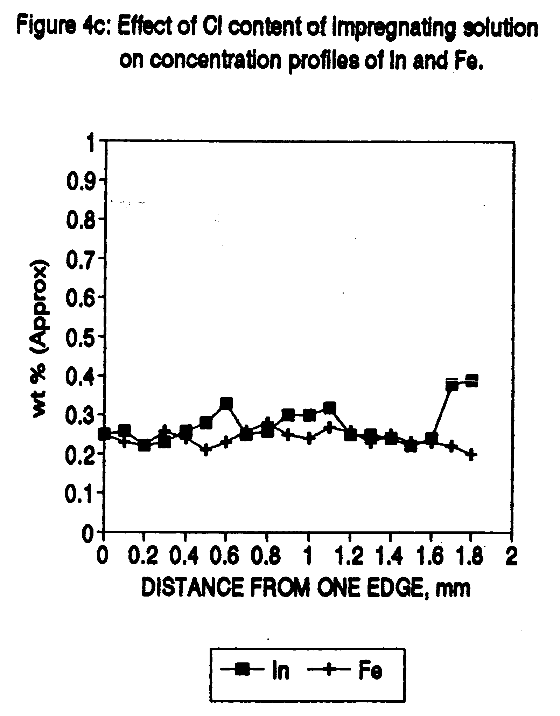

The alumina support was the same as that used for formulation A. These catalysts were prepared by the single step impregnation method (spraying) wherein the solution impregnated was a mixture of the solutions of all the active elements. The difference between these formulations was the quantity of acid anion Cl⁻ in the impregnating solution. This factor was varied by addition of HCl acid where required to maintain higher Cl⁻ concentration. The concentrations of this anion in the impregnating solution were, in the same order, 1, 3, 5, 7, 9 and 11 wt. %. The typical concentration profiles for Sn across the diameter of the catalyst sphere as determined by EDAX analysis are presented in Figure 4a. The results show that the distribution of Sn within the spatial geometry of the catalyst particle is affected by the quantity of Cl used in the impregnating solution. A minimum Cl content of 9 wt. %in the impregnating solution, is required for uniform distribution of Sn. Lower Cl content leads to higher concentration of Sn towards the shell of the spheroid. The other active elements are distributed uniformly. Typical EDAX concentration profiles for these are shown in figure 4b, for Pt, Sn and Cl and in figure 4c for In and Fe.

From Examples III and IV, it is seen that by judicious selection of the method of impregnation as well as by adjustment of the content of competing anion in the impregnating solution, the active metals can be distributed either uniformly or heterogeneously onto the support.

### EXAMPLE VIII

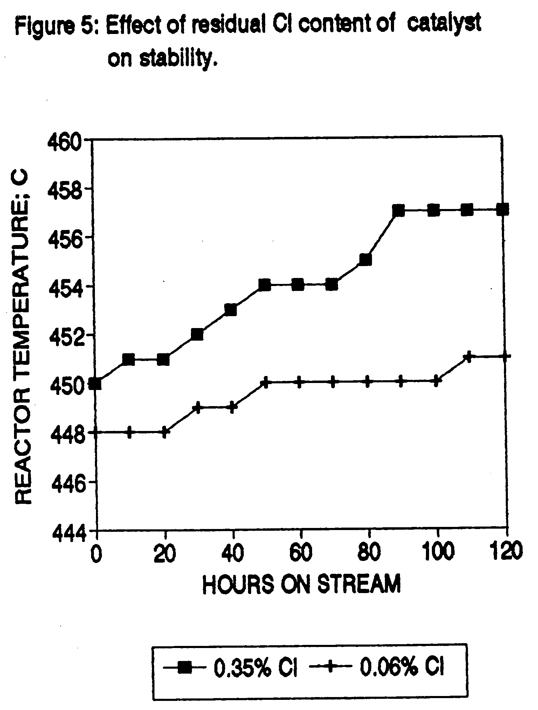

The effect of the quantity of residual acidic anion in the catalyst is shown through this example. The catalyst was prepared by the preferred method viz. two step impregnation. Hence, the formulation is essentially the same as that of A. The difference is in the residual chloride content of the catalyst. In one catalyst the residual chloride content is 0.35 wt. %, whereas in another it is 0.06 wt. %. The results of performance of these catalysts for the dehydrogenation of C₁₀-C₁₃ n-paraffins is presented in Figure 5. The results clearly show an inverse relation between stability and chloride content.

From the examples, it is clear that the invention enables the achievement of three distinctly different distributions of the active elements within the spatial geometry of the catalyst particle. For example, Formulation B of Example II exhibits uniform distribution of all the active elements Figure 4b, 4c. Formulation C of Example III exhibits restriction of Pt to only the shell region while the other active elements are uniformly distributed in the catalyst spheroid Figure 3. Formulations G to L of Example VII show a trend in the distribution of Sn with increasing Cl⁻ content of the impregnation solution [Figure 4a] therein. Thus, the present invention succeeds in establishing a method for obtaining a desired distribution of active elements within the catalyst particle.

Certain catalyst formulations prepared according to the present invention were tested for their activity, stability and selectivity for dehydrogenation of C₁₀-C₁₃ n-paraffins under the reaction conditions described hereafter. All experimental tests were performed in a once-through, tubular, axial flow, packed bed reactor operated at near isothermal conditions. The catalysts were reduced in a stream of hydrogen at 470°C for 2 hours in all the cases.

Essentially, two types of tests were carried out as follows:

Isothermal Tests: Monitoring of conversion of the paraffins under near isothermal reaction conditions. Specifically, the reaction conditions were:
- LHSV:: 30 h⁻¹,
- Temperature:: 450°C,
- Pressure:: atmospheric,
- H₂:HC (mol/mol):: 6.

Isoconversional Tests: Monitoring of the reactor temperature required for operating the reactor at a constant level of paraffin conversion viz 13% per pass conversion. The reaction conditions were:
- LHSV:: 20 h⁻¹
- Pressure:: 1.38 bar (20 psi)
- H₂:HC (mol/mol):: 6
- Conversion level:: 13 ± 0.5%.

As stated earlier, "conversion" is defined as "the moles of paraffin converted per mole of paraffin fed" and "selectivity" is defined as "the parts of monoolefins formed per part of paraffin converted across the reactor".

The scope of the patent is not restricted only to the examples as given above but is set forth in the claims.

## Claims

1. A novel catalyst composite for use in the dehydrogenation of paraffins to the corresponding monoolefins said composite incorporating within its spatial geometry on a percentage by weight basis a predetermined concentration gradient of the following active elements:
from 0.1 to 5.0% of a noble metal;
from 0.1 to 5.0 % of a metal of Group IV A;
from 0.1 to 6.0% of a metal of Group III A;
from 0.1 to 10.0 % of an alkali or alkaline earth metal element;
from 0.01 to 10.0% of a halogen; and
from 0.1 to 5.0 % of a metal of Group VIII selected from Fe, Co and Ni provided on a high surface area mesoporous support.

2. A novel catalyst as claimed in claim 1 wherein the catalyst composite consists of the following as active elements:
Platinum as the noble metal;
Tin (Sn) as the metal of Group IV A;
Indium (In) as the metal of Group III A;
Lithium (Li) as the alkali or alkaline earth metal element;
Chlorine (Cl) in combined form as the halogen; and
Iron (Fe) as the metal of Group VIII.

3. A novel catalyst as claimed in claim 2 wherein chlorine is present in an amount from 0.05% to 0.1% by weight and Fe is present in an amount of 0.2% by weight.

4. A novel catalyst as claimed in claim 2 wherein the mesoporous support is a spheroidal gamma alumina support having a diameter of 1.4 to 2.0 mm., a surface area in the range of 120 to 250 m²/g, a mesoporous pore distribution, a water adsorption capacity in the range of from 1.4 to 2.5 ml/g, a gamma crystallinity of from 60 to 80% and a bulk density of from 0.27 to 0.6 ml, preferably 0.3 gm/ml.

5. A novel catalyst as claimed in claim 4 wherein the support also comprises 0.1 to 5.0 wt. % Fe.

6. A process for the preparation of a novel catalyst composite incorporating a predetermined concentration gradient of active elements within its spatial geometry for use in the dehydrogenation of paraffins to the corresponding monoolefins which comprises incorporating on a percentage by weight basis within a high surface area mesoporous support:
from 0.1 to 5.0 % of a noble metal;
from 0.1 to 5.0 % of a metal of Group IV A;
from 0.1 to 6.0 % of a metal of Group III A;
from 0.1 to 10.0 % of an alkali or alkaline earth metal element;
from 0.01 to 10.0 % of a halogen; and
from 0.1 to 5.0 % of a metal of Group VIII selected from Fe, Co and Ni drying the composite in which said active elements have been incorporated; and
subjecting the dried composite to at least one calcination step, said mesoporous support being a spheroidal gamma alumina support having a diameter of 1.4 to 2.0 mm., a surface area in the range of 120 to 250 m²/g, a mesoporous pore distribution, a water adsorption capacity in the range of from 1.4 to 2.5 ml/g, a gamma crystallinity of from 60 to 80% and a bulk density of from 0.27 to 0.6 ml, preferably 0.3 gm/ml.

7. A process as claimed in any of the preceding claims wherein the active elements are incorporated into the said mesoporous support in a single step or stage wise by any conventional technique.

8. A process as claimed in claim 7 wherein the active elements are incorporated individually or in combination into the support during the sol stage of preparation of said support.

9. A process as claimed in claim 7 wherein the active elements are incorporated individually or in combination by impregnation of the finished support therewith.

10. A process as claimed in claim 8 wherein Fe is incorporated during the sol stage of preparation of the support or by impregnation into the finished support before, during or after incorporation of the other elements.

11. A process as claimed in claim 8 wherein Sn component is incorporated at the sol stage of preparation of the support prior to, along with or after the other active elements are incorporated preferably along with Pt or to the finished support.

12. A process as claimed in claim 9 wherein In is incorporated at the sol stage of preparation of the support or by impregnation into the finished product either simultaneously with the other elements or singly but preferably not prior to incorporation of Pt.

13. A process as claimed in claim 9 wherein Pt is incorporated by impregnation of the support either simultaneously with other elements or singly but preferably not after In.

14. A process as claimed in claim 9 wherein Li is incorporated at the sol stage in the preparation of the support or simultaneously with other elements or singly during impregnation of the support but preferably not after incorporation of the other elements.

15. A process as claimed in claim 14 wherein the impregnation is effected by employing an aqueous solvent, an organic solvent or mixture of the two in the presence of anions.

16. A process as claimed in claim 15 wherein anions are acidic anions preferably chloride anions in the range of 0.1 to 15.0% by weight preferably 8% to 10% by wt.

17. A process as claimed in any of claims 15 or 16 wherein the impregnating solution is heated to a temperature of from 40 to 70°C for a minimum of 30 minutes, cooled to a temperature in the range of 5°C to 40°C and maintained at this temperature during impregnation.

18. A process as claimed in claim 17 wherein the volume of said impregnating solution is 5% to 30% in excess of the water adsorption capacity of the support.

19. A process as claimed in claim 6 wherein Pt content is either uniformly distributed or restricted to a narrow shell region within the catalyst spheroid.

20. A process as claimed in claim 6 whereby the distribution of Sn within the spatial geometry of the catalyst particle may be varied from restriction to the shell region to uniform distribution throughout the particle, by the judicious control of chloride content of the impregnating solution.

21. A process for the preparation of a novel catalyst composite incorporating a predetermined concentration gradient of active elements within its spatial geometry for use in the dehydrogenation of paraffins to the corresponding monoolefins which comprises incorporating on a percentage by weight basis within a high surface area mesoporous support:
from 0.1 to 5.0 % of a noble metal;
from 0.1 to 5.0 % of a metal of Group IV A;
from 0.1 to 6.0% of a metal of Group III A;
from 0.1 to 10.0 % of an alkali or alkaline earth metal element;
from 0.01 to 10.0 % of a halogen; and
from 0.1 to 05.0 % of a metal of Group VIII selected from Fe, Co and Ni drying the composite in which said active elements have been incorporated; and
subjecting the dried composite to at least one calcination step;
subjecting further the calcined composite to controlled treatment with a solution of a salt of a weak base which on hydrolysis at elevated temperature releases NH₃, or with a solution of NH₄OH at between 40-70°C for 1 to 4 hours to decrease the chloride content to about 0.04 to 0.1 wt.%.

22. Use of a catalyst composite according to any one of claims 1 to 5 or obtained by the process claimed in any one of claims 6 to 21 in the dehydrogenation of paraffins to the corresponding monoolefins.

## Patentansprüche

1. Wir beanspruchen:
1. eine neue Katalysatorzusammensetzung zur Verwendung bei der Dehydrogenierung von Paraffinen zu den entsprechenden Monoolefinen, wobei die Zusammensetzung innerhalb ihrer räumlichen Geometrie auf einer Gewichtsanteilbasis einen bestimmten Konzentrationsgradienten der folgenden aktiven Elemente beinhaltet:
von 0,1 bis 5,0 % eines Edelmetalls;
von 0,1 bis 5,0 % eines Metalls der Gruppe IV A;
von 0,1 bis 6,0 % eines Metalls der Gruppe III A;
von 0,1 bis 10,0 % eines Alkali- oder Erdalkalimetallelements;
von 0,01 bis 10,0 % eines Halogens; und
von 0,1 bis 5,0 % eines Metalls der Gruppe VIII, ausgewählt aus Fe, Co und Ni, angeordnet auf einem mesoporösen Träger mit großer Oberfläche.

2. Neuer Katalysator gemäß Anspruch 1, wobei die Katalysatorzusammensetzung aus den folgenden als aktive Elemente besteht:
Platin als Edelmetall;
Zinn (Sn) als Metall der Gruppe IV A;
Indium (In) als Metall der Gruppe III A;
Lithium (Li) als das Alkali- oder Erdalkalimetallelement;
Chlor (Cl) in kombinierter Form als das Halogen; und
Eisen (Fe) als Metall der Gruppe VIII.

3. Neuer Katalysator gemäß Anspruch 2, wobei Chlor in einer Menge von 0,05 bis 0,1 Gew.-% vorliegt und Eisen in einer Menge von 0,2 Gew.-%.

4. Neuer Katalysator gemäß Anspruch 2, wobei der mesoporöse Träger ein sphäroidaler Gamma-Aluminium-Träger mit einem Durchmesser von 1,4 bis 2,0 mm ist, einer Oberfläche im Bereich von 120 bis 250 m²/g, einer mesoporösen Porenverteilung, einer Wasseradsorptionskapazität im Bereich von 1,4 bis 2,5 ml/g einer Gamma-Kristallinität von 60 bis 80 % und einer Schüttdichte von 0,27 bis 0,6 ml, bevorzugt 0,3 g/ml.

5. Neuer Katalysator gemäß Anspruch 4, wobei der Träger auch 0,1 bis 5,0 Gew.-% Fe umfaßt.

6. Verfahren zur Herstellung einer neuen Katalysatorzusammensetzung, die einen bestimmten Konzentrationsgradienten an aktiven Elementen innerhalb ihrer räumlichen Geometrie enthält, zur Verwendung bei der Dehydrogenierung von Paraffinen zu den entsprechenden Monoolefinen, wobei das Verfahren das Einbringen folgender Bestandteile auf Gew.-%-Basis in einen mesoporösen Träger mit hoher Oberfläche umfaßt:
von 0,1 bis 5,0 % eines Edelmetalls;
von 0,1 bis 5,0 % eines Metalls der Gruppe IV A;
von 0,1 bis 6,0 % eines Metalls der Gruppe III A;
von 0,1 bis 10,0 % eines Alkali- oder Erdalkalimetallelements;
von 0,01 bis 10,0 % eines Halogens; und
von 0,1 bis 5,0 % eines Metalls der Gruppe VIII, ausgewählt aus Fe, Co und Ni, Trocknen der Zusammensetzung in welche die aktiven Elemente eingebracht worden sind; und
Unterwerfen der getrockneten Zusammensetzung unter wenigstens einen Calcinierungsschritt, wobei der mesoporöse Träger ein sphäroidaler Gamma-Aluminium-Träger mit einem Durchmesser von 1,4 bis 2,0 mm ist, einer Oberfläche im Bereich von 120 bis 250 m²/g, einer mesoporösen Porenverteilung, einer Wasseradsorptionskapazität im Bereich von 1,4 bis 2,5 ml/g, einer Gamma-Kristallinität von 60 bis 80 % und einer Schüttdichte von 0,27 bis 0,6 mm, bevorzugt 0,3 g/ml.

7. Verfahren gemäß einem der vorherigen Ansprüche, wobei die aktiven Elemente in einem Einzelschritt oder stufenweise durch irgendein herkömmliches Verfahren in den mesoporösen Träger eingebracht werden.

8. Verfahren gemäß Anspruch 7, wobei die aktiven Elemente einzeln oder in Kombination während der Solstufe der Herstellung des Trägers in denselben eingebracht werden.

9. Verfahren gemäß Anspruch 7, wobei die aktiven Elemente einzeln oder in Kombination durch Imprägnieren des fertiggestellten Trägers mit denselben eingebracht werden.

10. Verfahren gemäß Anspruch 8, wobei Eisen während der Solstufe der Herstellung des Trägers oder durch Imprägnieren des fertiggestellten Trägers vor, während oder nach dem Einbringen der anderen Elemente eingebracht wird.

11. Verfahren gemäß Anspruch 8, wobei der Sn-Bestandteil während der Solstufe der Herstellung des Trägers vor, gleichzeitig mit oder nachdem die anderen Elemente eingebracht wurden, eingebracht wird, bevorzugt zusammen mit Pt oder in den fertiggestellten Träger.

12. Verfahren gemäß Anspruch 9, wobei In auf der Solstufe der Herstellung des Trägers oder durch Imprägnieren des fertiggestellten Produkts, entweder gleichzeitig oder mit den anderen Elementen oder einzeln, aber bevorzugt nicht vor dem Einbringen von Pt, eingebracht wird.

13. Verfahren gemäß Anspruch 9, wobei Pt durch Imprägnieren des Trägers, entweder gleichzeitig mit anderen Elementen oder einzeln, aber bevorzugt nicht nach In, eingebracht wird.

14. Verfahren gemäß Anspruch 9, wobei Li auf der Solstufe bei der Herstellung des Trägers oder gleichzeitig mit anderen Elementen oder einzeln während des Imprägnierens des Trägers eingebracht wird, aber bevorzugt nicht nach dem Einbringen der anderen Elemente.

15. Verfahren gemäß Anspruch 14, wobei das Imprägnieren durch Einsatz eines wässrigen Lösungsmittels, eines organischen Lösungsmittels oder einer Mischung der zwei in Gegenwart von Anionen bewirkt wird.

16. Verfahren gemäß Anspruch 15, wobei Anionen acidische Anionen sind, bevorzugt Chloridanionen im Bereich von 0,1 bis 15 Gew.-%, bevorzugt 8 bis 10 Gew.-%.

17. Verfahren gemäß einem, der Ansprüche 15 oder 16, wobei die Imprägnierlösung auf eine Temperatur von 40 bis 70 °C für ein Minimum von 30 Minuten erwärmt wird, auf eine Temperatur im Bereich von 5 bis 40 °C gekühlt wird und während des Imprägnierens bei dieser Temperatur gehalten wird.

18. Verfahren gemäß Anspruch 17, wobei das Volumen der Imprägnierlösung einen Überschuss von 5 bis 30 % hinsichtlich der Wasseradsorptionskapazität des Trägers umfaßt.

19. Verfahren gemäß Anspruch 6, wobei der Platingehalt entweder einheitlich verteilt oder auf einen schmalen Mantelbereich innerhalb des Katalysatorsphäroids beschränkt ist.

20. Verfahren gemäß Anspruch 6, wobei die Verteilung von Zinn innerhalb der räumlichen Geometrie des Katalysatorpartikels von einer Beschränkung auf den Hüllbereich bis zu einer einheitlichen Verteilung durch das Partikel hindurch durch vernünftige Steuerung des Chloridgehalts der Imprägnierlösung variiert werden kann.

21. Verfahren zur Herstellung einer neuen Katalysatorzusammensetzung durch Einbringen eines bestimmten Konzentrationsgradienten an aktiven Elementen innerhalb dessen räumlicher Geometrie zur Verwendung bei der Dehydrogenierung von Paraffinen zu den entsprechenden Monoolefinen, wobei das Verfahren das Einbringen folgender Bestandteile auf Gew.-%-Basis in einen mesoporösen Träger mit großer Oberfläche umfaßt:
von 0,1 bis 5 % eines Edelmetalls;
von 0,1 bis 5,0 % eines Metalls der Gruppe IV A;
von 0,1 bis 6,0 % eines Metalls der Gruppe III A;
von 0,1 bis 10,0 % eines Alkali- oder Erdalkalimetallelements;
von 0,01 bis 10,0 % eines Halogens; und
von 0,1 bis 5,0 % eines Metalls der Gruppe VIII, ausgewählt aus Fe, Co und Ni, Trocknen der Zusammensetzung in welche die aktiven Elemente eingebracht worden sind; und
Unterwerfen der getrockneten Zusammensetzung unter wenigstens einen Calcinierungsschritt;
weiterhin Unterwerfen der calcinierten Zusammensetzung einer kontrollierten Behandlung mit einer Lösung eines Salzes einer schwachen Base, welche auf Hydrolyse bei erhöhten Temperaturen NH₃ freisetzt, oder mit einer Lösung von NH₄OH zwischen 40 bis 70 °C für 1 bis 4 Stunden, um den Chloridgehalt auf ungefähr 0,04 bis 0,1 Gew.-% herabzusetzen.

22. Verwendung einer Katalysatorzusammensetzung gemäß einem der Ansprüche 1 bis 5, oder erhalten durch das Verfahren gemäß einem der Ansprüche 6 bis 21, bei der Dehydrogenierung von Paraffinen zu den entsprechenden Monoolefinen.

## Revendications

1. Nouveau composite catalytique destiné à être utilisé dans la déshydrogénation de paraffines en les monooléfines correspondantes, ledit composite incorporant dans sa géométrie spatiale sur une base de pourcentage en masse un gradient de concentration prédéterminé des éléments actifs suivants :
de 0,1 à 5,0 % d'un métal noble ;
de 0,1 à 5,0 % d'un métal du groupe IV A ;
de 0,1 à 6,0 % d'un métal du groupe III A ;
de 0,1 à 10,0 % d'un élément de type métal alcalin ou alcalino-terreux ;
de 0,01 à 10,0 % d'un halogène ; et
de 0,1 à 5,0 % d'un métal du groupe VIII choisi parmi Fe, Co et Ni disposé sur un support mésoporeux de grande aire spécifique.

2. Nouveau catalyseur selon la revendication 1, dans lequel le composite catalytique consiste en ce qui suit comme éléments actifs :
platine comme métal noble ;
étain (Sn) comme métal du groupe IV A ;
indium (In) comme métal du groupe III A ;
lithium (Li) comme élément de type métal alcalin ou alcalino-terreux ;
chlore (Cl) sous forme combinée comme halogène ; et
fer (Fe) comme métal du groupe VIII.

3. Nouveau catalyseur selon la revendication 2, dans lequel le chlore est présent en une quantité de 0,05 % à 0,1 % en masse et Fe est présent en une quantité de 0,2 % en masse.

4. Nouveau catalyseur selon la revendication 2, dans lequel le support mésoporeux est un support d'alumine gamma sphéroïdale ayant un diamètre de 1,4 à 2,0 mm, une aire spécifique dans le domaine de 120 à 250 m²/g, une distribution de pores mésoporeuse, une capacité d'adsorption d'eau dans le domaine de 1,4 à 2,5 ml/g, une cristallinité gamma de 60 à 80 % et une masse volumique apparente de 0,27 à 0,6 ml, de préférence de 0,3 g/ml.

5. Nouveau catalyseur selon la revendication 4, dans lequel le support comprend aussi 0,1 à 5,0 % en masse de Fe.

6. Procédé de préparation d'un nouveau composite catalytique incorporant un gradient de concentration prédéterminé d'éléments actifs dans sa géométrie spatiale destiné à être utilisé dans la déshydrogénation de paraffines en les monooléfines correspondantes qui comprend l'incorporation sur une base de pourcentage en masse dans un support mésoporeux de grande aire spécifique :
de 0,1 à 5,0 % d'un métal noble ;
de 0,1 à 5,0 % d'un métal du groupe IV A ;
de 0,1 à 6,0 % d'un métal du groupe III A ;
de 0,1 à 10,0 % d'un élément de type métal alcalin ou alcalino-terreux ;
de 0,01 à 10,0 % d'un halogène ; et
de 0,1 à 5,0 % d'un métal du groupe VIII choisi parmi Fe, Co et Ni, le séchage du composite dans lequel lesdits éléments actifs ont été incorporés ; et
la soumission du composite séché à au moins une étape de calcination, ledit support mésoporeux étant un support d'alumine gamma sphéroïdale ayant un diamètre de 1,4 à 2,0 mm, une aire spécifique dans le domaine de 120 à 250 m²/g, une distribution de pores mésoporeuse, une capacité d'adsorption d'eau dans le domaine de 1,4 à 2,5 ml/g, une cristallinité gamma de 60 à 80 % et une masse volumique apparente de 0,27 à 0,6 ml, de préférence de 0,3 g/ml.

7. Procédé selon l'une quelconque des revendications précédentes, dans lequel les éléments actifs sont incorporés dans ledit support mésoporeux en une seule étape ou par étapes par toute technique conventionnelle.

8. Procédé selon la revendication 7, dans lequel les éléments actifs sont incorporés individuellement ou en combinaison dans le support au stade sol de la préparation dudit support.

9. Procédé selon la revendication 7, dans lequel les éléments actifs sont incorporés individuellement ou en combinaison par imprégnation du support fini avec ceux-ci.

10. Procédé selon la revendication 8, dans lequel Fe est incorporé au stade sol de la préparation du support ou par imprégnation dans le support fini avant, pendant ou après l'incorporation des autres éléments.

11. Procédé selon la revendication 8, dans lequel le composant Sn est incorporé au stade sol de la préparation du support avant, pendant ou après l'incorporation des autres éléments actifs, de préférence en même temps que Pt, ou au support fini.

12. Procédé selon la revendication 9, dans lequel In est incorporé au stade sol de la préparation du support ou par imprégnation du produit fini en même temps que les autres éléments ou isolément, mais de préférence pas avant l'incorporation de Pt.

13. Procédé selon la revendication 9, dans lequel Pt est incorporé par imprégnation du support en même temps que les autres éléments ou isolément, mais de préférence pas après In.

14. Procédé selon la revendication 9, dans lequel Li est incorporé au stade sol de la préparation du support ou en même temps que les autres éléments ou isolément pendant l'imprégnation du support, mais de préférence pas après l'incorporation des autres éléments.

15. Procédé selon la revendication 14, dans lequel l'imprégnation est réalisée au moyen d'un solvant aqueux, d'un solvant organique ou d'un mélange des deux en présence d'anions.

16. Procédé selon la revendication 15, dans lequel les anions sont des anions acides, de préférence des anions chlorure dans le domaine de 0,1 à 15,0 % en masse, de préférence de 8 % à 10 % en masse.

17. Procédé selon l'une quelconque des revendications 15 et 16, dans lequel la solution d'imprégnation est chauffée à une température de 40 à 70°C pendant un minimum de 30 minutes, refroidie à une température dans le domaine de 5°C à 40°C et maintenue à cette température pendant l'imprégnation.

18. Procédé selon la revendication 17, dans lequel le volume de ladite solution d'imprégnation dépasse de 5 % à 30 % la capacité d'adsorption d'eau du support.

19. Procédé selon la revendication 6, dans lequel la teneur en Pt est distribuée uniformément ou limitée à une région d'enveloppe étroite dans le sphéroïde de catalyseur.

20. Procédé selon la revendication 6, dans lequel la distribution de Sn dans la géométrie spatiale de la particule de catalyseur peut être amenée à varier depuis la limitation à la région d'enveloppe jusqu'à la distribution uniforme dans toute la particule, par le contrôle judicieux de la teneur en chlorure de la solution d'imprégnation.

21. Procédé de préparation d'un nouveau composite catalytique incorporant un gradient de concentration prédéterminé d'éléments actifs dans sa géométrie spatiale destiné à être utilisé dans la déshydrogénation de paraffines en les monooléfines correspondantes qui comprend l'incorporation sur une base de pourcentage en masse dans un support mésoporeux de grande aire spécifique :
de 0,1 à 5,0 % d'un métal noble ;
de 0,1 à 5,0 % d'un métal du groupe IV A ;
de 0,1 à 6,0 % d'un métal du groupe III A ;
de 0,1 à 10,0 % d'un élément de type métal alcalin ou alcalino-terreux ;
de 0,01 à 10,0 % d'un halogène ; et
de 0,1 à 5,0 % d'un métal du groupe VIII choisi parmi Fe, Co et Ni, le séchage du composite dans lequel lesdits éléments actifs ont été incorporés ; et
la soumission du composite séché à au moins une étape de calcination ;
en outre la soumission du composite calciné à un traitement contrôlé avec une solution d'un sel d'une base faible qui, par hydrolyse à température élevée, libère NH₃, ou avec une solution de NH₄OH à 40-70°C pendant 1 à 4 h pour abaisser la teneur en chlorure à environ 0,04 à 0,1 % en masse.

22. Utilisation d'un composite catalytique selon l'une quelconque des revendications 1 à 5 ou obtenu par le procédé selon l'une quelconque des revendications 6 à 21 dans la déshydrogénation de paraffines en les monooléfines correspondantes.
